Europäisches Patentamt

**European Patent Office**

Office européen des brevets

(19)

(11) Publication number: **0 149 254**
**A2**

(12) # EUROPEAN PATENT APPLICATION

(21) Application number: **84116465.0**

(22) Date of filing: **28.12.84**

(51) Int. Cl.⁴: **A 61 K 37/02**
**A 61 K 35/24**

(30) Priority: **29.12.83 US 566515**

(43) Date of publication of application:
**24.07.85 Bulletin 85/30**

(84) Designated Contracting States:
**AT BE CH DE FR GB IT LI LU NL SE**

(71) Applicant: **THE RESEARCH FOUNDATION OF STATE UNIVERSITY OF NEW YORK**
**PO Box 9**
**Albany New York 12201(US)**

(72) Inventor: **Pollack, Jerry J.**
**10 Westminster Court Nesconset**
**N.Y. 11767(US)**

(72) Inventor: **MacKay, Bruce J.**
**863 Robin Road Somerville**
**N.J. 08876(US)**

(74) Representative: **Wager, Peter Dr. Dipl.-Chem.**
**Morassistrasse 8/II**
**D-8000 Munchen 5(DE)**

(54) Fungicidal polypeptide compositions containing L-histidine.

(57) Polypeptides containing a substantial proportion of L-histidine are effective fungicidal agents. They are particularly effective against *S. mutans* and *C. albicans* and have a high degree of safety and nontoxicity because of their structural similarity to naturally occurring histidine-rich polypeptides which are unique to the salivas of humans and old world monkeys.

FIG. I

# FUNGICIDAL POLYPEPTIDE COMPOSITIONS CONTAINING L-HISTIDINE

## BACKGROUND OF THE INVENTION

Fungal infections of mucosal tissue are particularly troublesome because of the difficulty of effective administration of fungistatic or fungicidal agents, since systemic administration of such agents is not desirable and usually not effective. On the other hand, because of the normal fluid excretion from the mucosa there is a substantial tendency of these fluid excretions to wash out topically administered therapeutic agents giving rise to the need for either high levels of administration which may lead to undesirable side effects or frequent administration which is not always possible and not always adhered to by the patient. It is therefore desirable to seek fungistatic and fungicidal agents which when used in effective dosages overcome these problems.

There are three areas of concern to physicians and dentists, all involving a different problem of fungal infection. Under normal circumstances fungal infection of the oral cavity is not a problem since salivary clearance and oral hygiene, on a regular basis will not permit fungal infections to take hold. A major and important exception to this situation occurs among denture wearers. In 1956, Fisher (J.Prosthet.Dent., 6, 593 (1956)) proposed that a connection might exist between poor denture hygiene and denture stomatitis. This relationship was demonstrated by Budtz-Jorgensen and Bertram (Acta.Ondontol.Scand., 28, 71 (1970)). Further work by Budtz-Jorgensen (J.A.D.A., 96, 474 (1978)), shows that Candida albicans and related species play a major role in initiating, maintaining and aggravating the disease. He found, for example, that denture stomatitis is present in about 65% of the wearers of complete dentures in the Danish community. This work was confirmed by Tarbet in a United States study (J.Prothet.Dent., 48, 647 (1982)).

A recent survey of this problem was published by Budtz-Jorgensen as a review (Scand.J.Dent.Res., 82, 151-190 (1974)). In his review, at page 174, Budtz-Jorgensen discusses host defense mechanisms in Candida induced denture stomatitis and indicates that certain substances in saliva, for example, lysozyme, act to retard the growth of bacteria and fungi. However, he states (at page 175) that dentures prevent salivary anti-candidal substances from getting into contact with and combatting the flora propagating on the palatal mucosal surface. It has been suggested that C. albicans must be eliminated from the acrylic surface of the denture itself in order to halt the disease process (Davenport, Br.Dent.J., 129, 151 (1970)). These factors are critical to the design of antifungal agents for denture wearers since they must not only be active but also they must be effectively delivered and/or localized at the interface between the denture and the gum.

It has been previously reported that cationic histidine-rich polypeptides are present in human parotid saliva (Balekjian, Biochem.Biophys.Res.Com., 50, 676 (1973);Baum, et al.,Arch.Biochem.Biophys., 177, 427 (1976); Peters and Azen, Biochem.Genet.,15, 925 (1977); and Peters,et.al., Biochem.Genet., 15, 947 (1977)). While several different histidine-rich polypeptides,(hereinafter HRP) derived from the salivary glands have been reported and some have been partially sequenced, pure preparations have not heretofore been provided. Furthermore, to date, a biological role for the HRP has not been documented. As outlined in this application, the HRP, as well as poly-L-histidine and synthetic L-histidine peptides, exhibit antimicrobial actions.

Although Yphantis, et.al., (J.Bacteriol., 94, 1509 (1967) have pointed out that Candida utilis is sensitive to the cationic polypeptide, poly-L-lysine, which is also antibacterial (Katchalski, et al., The Proteins II, 405, 1964), poly-L-lysine unfortunately is known to have unacceptable levels of toxicity in mammalian systems (see for example Rubini, et al., Proc.Soc.Exptl.Biol.Med., 82; 231 (1953)).

4

Other mucosal areas where *Candida* infections are prevalent are the vaginal and urethral mucosa. At the present time, two antifungal agents are known which are clinically used with a reasonable measure of success, these are nystatin and clotrimazole. However, since synthetic fungicides exhibit undesirable toxic side-effects, it would be preferable to use either the body's own naturally produced fungicides or agents structurally related to these natural body products. Naturally occurring agents may also serve to reduce the possibility of survival of resistant forms of the fungi. While fungal infection of the female is more common, current therapeutic methods also consider treatment of the male urethral mucosa, it would be desirable to provide compounds compatible therewith.

## SUMMARY OF THE INVENTION

Certain histidine-rich polypeptides (polymers) have been purified from human parotid saliva, It has been found that those HRP having a substantial proportion, i.e., between about 14 and about 40 mol % amino acid residues of L-histidine, have antibacterial and antifungal properties, in particular, against Streptococcus mutans and Candida albicans.

These microbiocidal properties reside in the chemical structural characteristics of the histidine residue. However, the amino acid, histidine (monomer) does not possess these properties and the lower histidine oligomers have a lower level of effectiveness. Useful properties begin to appear at the tetramer level and the L-histidine heptamer has been shown to be highly effective. Thus, oligomers of, say, 4 to 10 units are desirable. However, poly-L-histidine having more than 10 units in the sequence is also active and should be considered within the purview of the present invention.

The polypeptides of the present invention are administrable to the loci of infection, in particular, the oral, vaginal and urethral mucosal surfaces. Delivery may be by any conventional means, preferably topical means. In the case of oral administration, this would include dentrifrices, mouth washes, denture washes or soaks and denture adhesives or cements. Incorporation into polymers associated within the denture, in particular, with the interface of the denture with the gum, should be considered as part of the invention.

Vaginal administration may be by the usual carriers such as douches, foams, creams, and suppositories, the longer lasting forms being preferred.

Urethral administration is preferably by creams which may be of the same or similar formulation to that used for vaginal administration.

## DESCRIPTION OF THE PREFERRED EMBODIMENTS

The active components of the present invention are polypeptides which are at least 4 peptide moieties long and which contain at least 14 mol % amino acid residues of L-histidine, there is no upper limit. Activity has been found with a tetramer of L-histidine; however, this level is not deemed entirely satisfactory for clinical purposes. A heptamer of L-histidine has been found to yield very satisfactory results.

With respect to the naturally occurring peptides, peptides having as low as 14% residues of L-histidine have been found active, although better results are obtained with those peptides having 20% or greater suitably 24 to 35% residues of L-histidine. While the reasons are not fully understood, it would appear that a peptide of less than 4 units will penetrate the cell membrane of the microbe which it is attacking and be disposed of by the internal enzymes. On the other hand, units exceeding this size will be blocked by the cell membrane and work their microbiocidal effect thereon. It is not essential that there be a predetermined number of sequential L-histidine units provided that the minimum chain length and the minimum percent histidine residue conditions are met.

Poly-L-histidine is a commercially available mixture of histidine chains of various lengths produced by the polymerization of N-carboxy anhydride of histidine in the presence of a primary or secondary amine.

The histidine oligomers are similarly commercially available and are produced by single unit build-up in accordance with standard peptide synthesis methodologies.

The modes of administration are those well recognized in the art for treatment or prevention of the bacterial or fungal infections of the mucosa. Thus, for example, there may be provided vaginal creams, suppositories or solutions comprising between 0.2 and 2% by weight of the histidine containing material. Where the infection is an external one, the cream may be gently massaged into the surrounding areas twice daily. When intravaginal use is recommended, approximately 5 grams of the cream should be injected using a conventional applicator high into the vaginal vault once or twice a day with administration continued for about 1 to about 4 weeks. It may be preferred to utilize vaginal suppositories which are similarly inserted high into the vaginal vault once or twice daily and treatment continued for the same period of time.

The histidines peptides may also be incorporated into vaginal douches, however, it should be borne in mind that for the treatment or prevention of vaginal infections continual and frequent administration is desirable, while in most cases douching on a once or twice daily frequency for from one week to four weeks is usually contraindicated for other reasons.

A conventional denture adhesive paste may be formulated containing from about 12.5 to about 1,500 milligrams of histidine peptide materials per approximately 100 grams of paste if about 2 grams of this paste is applied in the conventional manner to the contact surface of the denture prior to insertion into the mouth. Such application should be made after overnight soaking in the denture cleanser. Denture cleansers may be formulated by the addition of between 6 and 720, suitably about 60 to 240 milligrams of active agent in a tablet of 3 to 3.5 grams. Such a tablet is dissolved in approximately 250 ml. of water yielding, at a concentration of 240 mg. per tablet, approximately 1 milligram per ml. of active material. In the preferred mode of use, the denture, after removal from the patient's mouth, is soaked in this cleanser for from about 8 to about 12 hours. It is not necessary, indeed it is preferred not to rinse the denture prior to insertion.

If desired, in place of utilizing a denture cement, some denture wearers prefer to use a denture adhesive powder which contains between about 12.5 to about 1,500 milligrams per about 100 grams of powder of which from about 1 to about 2 grams are sprinkled onto the gum contact surface of the denture after overnight soaking and prior to insertion into the mouth.

A mouth spray containing between about 2.5 and about 300 milligrams of the histidine peptide material per about 100 ml. of spray may be formulated. This material may be sprayed as an antibacterial agent in 0.25 to 0.5 ml. aliquots onto the tooth and gingiva surfaces of each quadrant between 1 and 3 times per day. In the case of denture wearers, the spray may be utilized directly on the denture surface prior to daily insertion of the denture.

If desired a mouthwash formulation may be provided containing between about 25 to about 3,000 milligrams of histidine peptide material per 1,000 ml. of mouthwash and similarly a toothpaste may be formulated containing between about 2.5 and 500 mg. per about 75 grams of toothpaste.

## BRIEF DESCRIPTION OF THE DRAWINGS

Figure 1 is a plot showing the effect of the salivary histidine-rich polypeptides on the growth of C. albicans.

Figure 2 is a plot showing the effect of the salivary histidine-rich polypeptides on the viability of three strains of C. albicans under non-growing conditions.

Figure 3 is a plot of optical density against incubation time of the effect of HRP 7 and a control on the growth of C. albicans.

Figure 4 is a plot of optical density against incubation time of the effect of tetra L-histidine, hepta-L-histidine, and a control on the growth of C. albicans.

## EXAMPLE I

### Isolation and Characterization of HRPs

The HRPs are obtained from the parotid saliva of healthy donors whose salivary flow is stimulated by 2% citric acid or sour lemon drops. An enriched preparation of the HRPs free from other salivary components may be carried out by fractionation on Sephadex G25. A small 280 nm absorption peak which contained a significant amount of 227 nm absorbing material eluted from the column immediately following the voiding peak and prior to the salt peak. This enriched fraction is again fractionated on Sephadex G-25 to eliminate minor amounts of other salivary components. The individual components of the mixture may be separated either by high performance liquid chromatography or by cationic polyacrylamide gel electrophoresis (cationic page), in accordance with the method described by Baum,et.al.,(J.Dent.Res., 56, 1115 (1977)). In the modification of the procedure slab gels were used instead of disc gels. Tables 1, 2 and 3 provide data on the amino acid compositions of the histidine-rich polypeptides.

TABLE I

Histidine content of the human parotid histidine-rich polypeptides isolated by cationic polyacrylamide gel electrophoresis and High Performance Liquid Chromatography

| Polypeptide | Percentage of Histidine[a] |
|-------------|------------------|
| HRP 1 | 17.8[b] |
| HRP 2 | 13.8[b] |
| HRP 3 | 24.2[b] |
| HRP 4 | 25.2[b] |
| HRP 5 | 33.7[b] |
| HRP 6 | 35.0[b] |
| HRP 6a, 6b, 6c | 26.5[c] |
| HRP 7 | 26.8[c] |

[a] Based on the number of residues per 100 residues from 24 h 6N HCl hydrolysates of isolated histidine-rich polypeptides. Results express the mean of two determinations.

[b] All percentage values are calculated on the total number of amino acid residues except for glycine. Control polyacrylamide when electrophoresed and extracted by the method of Gibson and Gracy, Anal. Biochem., 96, 352 (1979), yield percent amino acid values of glycine which significantly exceed the expected values.

[c] From the preparations isolated by high performance liquid chromatography, glycine was omitted from the calculations for comparative purposes.

Table 2

Amino Acid Compositions of HPLC Fractions[2]

| Amino Acid | P HRPs1,2 | 1 HRPs3,4 | J HRPs5,6 | H HRPs6a,b,c | F HRP-7 |
|---|---|---|---|---|---|
| Asp | 13.6 | 12.5 | 4.9 | 4.2 | 8.3 |
| Thr | 0.0 | 0.0 | 0.0 | 0.0 | 0.0 |
| Ser | 7.1 | 9.2 | 8.0 | 6.7 | 7.5 |
| Glu | 9.3 | 4.6 | 4.7 | 7.0 | 1.9 |
| Pro | 4.3 | Tr | Tr | 0.0 | 0.6 |
| Gly | 10.1 | 7.2 | 8.9 | 8.4 | 10.2 |
| Ala | Tr | 2.1 | 3.2 | 3.5 | 7.7 |
| Cys | 0.0 | 0.0 | 0.0 | 0.0 | 0.0 |
| Val | 0.0 | 0.0 | 0.0 | 0.0 | 0.0 |
| Met | 0.0 | 0.0 | 0.1 | 0.0 | 0.0 |
| Ile | 0.0 | 0.0 | 0.0 | 0.0 | 0.0 |
| Leu | 3.0 | 3.2 | 0.3 | 0.0 | 0.2 |
| Tyr | 12.4 | 11.6 | 8.4 | 2.1 | 0.5 |
| Phe | 9.4 | 3.9 | 3.7 | 4.2 | 0.4 |
| Trp | ND | ND | ND | ND | ND |
| His | 15.9 | 19.8 | 27.8 | 24.3 | 24.1 |
| Lys | 6.9 | 11.9 | 15.3 | 16.8 | 21.6 |
| Arg | 8.2 | 14.1 | 14.2 | 10.3 | 17.0 |

[a]Data expressed as residues/100 residues.

## Table 3

Amino Acid Compositions of the Histidine-Rich
Polypeptides Isolated by Polyacrylamide Gel
Electrophoresis

| Amino Acid | HRP-3 | HRP-4 | HRP-5 | HRP-6 |
|---|---|---|---|---|
| Asp | 15.8 | 14.1 | 6.0 | 3.1 |
| Thr | 0.0 | 0.0 | 0.0 | 0.0 |
| Ser | 11.7 | 10.2 | 10.4 | 8.7 |
| Glu | 4.8 | 5.0 | 5.5 | 7.1 |
| Pro | Tr | Tr | 0.0 | 0.0 |
| Gly | ND | ND | ND | ND |
| Ala | 2.8 | 1.9 | 4.1 | 2.0 |
| Cys | 0.0 | 0.0 | 0.0 | 0.0 |
| Val | 0.0 | 0.0 | 0.0 | 0.0 |
| Met | 0.0 | 0.0 | 0.0 | 0.0 |
| Ile | 0.0 | 0.0 | 0.0 | 0.0 |
| Leu | 3.2 | 3.6 | 0.0 | 0.0 |
| Tyr | 7.6 | 6.7 | 2.4 | 3.0 |
| Phe | 3.4 | 4.0 | 4.7 | 5.1 |
| Trp | ND | ND | ND | ND |
| His | 24.2 | 25.2 | 33.7 | 35.0 |
| Lys | 11.8 | 13.4 | 17.4 | 19.1 |
| Arg | 14.8 | 15.7 | 15.9 | 16.8 |

## EXAMPLE II

### Biological Properties of the HRP and Poly-L-Histidine

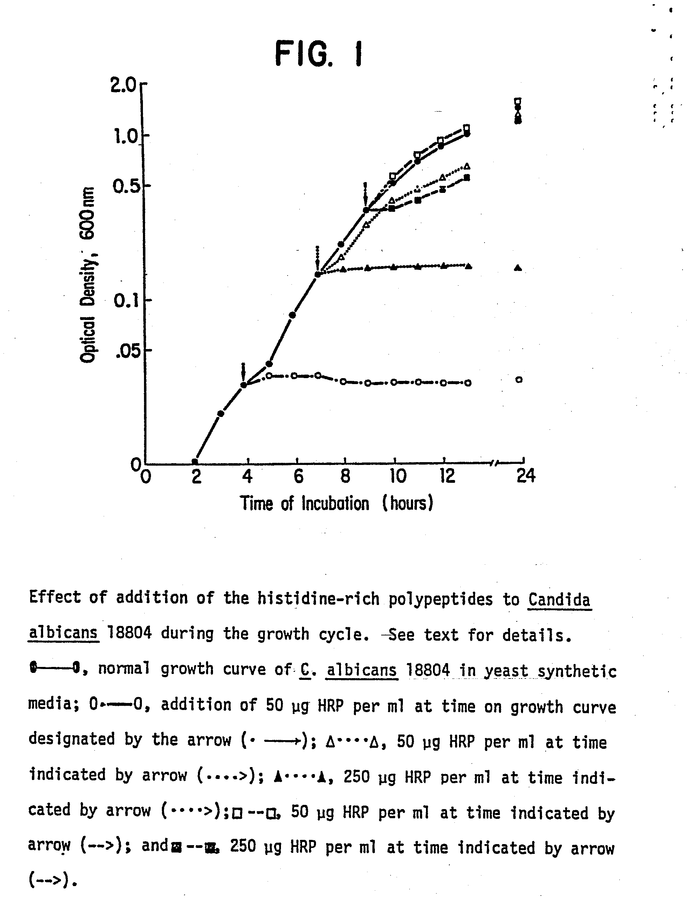

Table 4 demonstrates that at concentrations of 25 ug of mixture HRP 1-7 per ml or higher in the yeast synthetic media, there was virtually complete inhibition of growth after a 24 hour incubation period. At 10 $\mu$g HRP per ml, a slight inhibition was noted. When HRP was not placed in the growth medium initially but was added to growing cells, the following observations were noted (Fig. 1). (i) The inhibitory effect on growth was greatest at lower cell densities, (ii) the higher the cell density, the greater the concentration required to inhibit growth, (iii) complete growth inhibition could be obtained for 24 hours at cell densities of approximately $10^6$ colony forming units per ml (optical density 600 nm of 0.2) using an HRP concentration of 250 $\mu$g per ml (Fig. 1), (iv) inhibition of growth at this cell concentration ($10^6$ colony forming units per ml) by 50 $\mu$g per ml was delayed and was not complete as cells reached optical densities of the control after a 24 hour period, and (v) at still higher cell densities, there was no inhibition of growth at 50 $\mu$g HRP per ml but there was the delay in growth at 250 $\mu$g HRP per ml. Poly-L-histidine was found to be similarly effective to the HRP (data not shown).

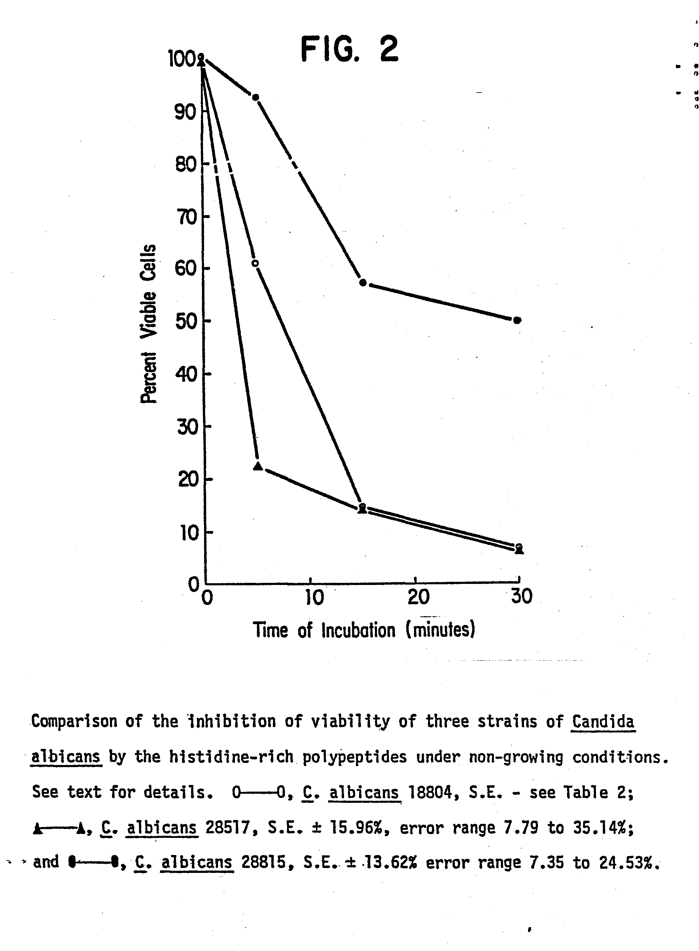

Under growing conditions, loss of viability of C. albicans 18804 correlated with inhibition of growth (Table 4). At concentrations of 25 $\mu$g HRP per ml or higher, greater than 99% killing of the yeast was observed when cells were plated after a period of 24 hours in the growth media. Under non-growing conditions, greater than 90% inhibition of viability of C. albicans 18804 was noted with the tested concentration of 100 $\mu$g per ml HRP after a period of 30 min. (Table 5). Compared to C. albicans 28517 was more sensitive while C. albicans 18804, C. albicans 28517 was more sensitive while C. albicans was considerably more resistant under these experimental conditions (Fig. 2).

Table 4

Effect of the Histidine-Rich Polypeptides on the Growth and Viability of Candida albicans 18804

| HRP concn. (ug/ml) | O.D. 600 nm[a] | CFU[b] | Percent loss of Viability[c] |
|---|---|---|---|
| 0 (Control) | 1.4 | $10^7$ | ---- |
| 5 | 1.4 | $10^7$ | 0 |
| 10 | 1.2 | $10^7$ | 0 |
| 25 | 0.035 | $14 \times 10^3$ | 99.86 |
| 50 | 0.018 | $73.6 \times 10^2$ | 99.93 |
| 75 | 0.013 | $12.4 \times 10^3$ | 99.98 |
| 100 | 0.01 | $73.6 \times 10^2$ | 99.93 |
| 150 | 0.01 | $5.3 \times 10^2$ | 99.99 |
| 250 | 0.005 | $11.1 \times 10^2$ | 99.99 |

[a] The optical density at 600 nm was measured after 24 hours growth at 37°C in the yeast synthetic media.

[b] Colony-forming units per ml were determined after plating aliquots from 24 hour growth cultures onto yeast morphology agar for an additional 48 hours at 37°C.

[c] Expressed as a percentage of the control.

Table 5

Effect of the Histidine-Rich Polypeptides on the Viability of <u>Candida albicans</u> 18804 under non-growing Conditions

| Time of Incubation (min)[a] | Control | HRP exposed[b] | Percent Loss of Viability[c] |
|---|---|---|---|
| | CFU[b] | | |
| 0 | $9.6 \times 10^5 \pm 1.3 \times 10^5$ | $11.4 \times 10^5 \pm 1.0 \times 10^5$ | ---- |
| 5 | $9.0 \times 10^5 \pm 1.4 \times 10^5$ | $5.4 \times 10^5 \pm 0.6 \times 10^5$ | 39.9 |
| 15 | $12.9 \times 10^5 \pm 1.6 \times 10^5$ | $18.1 \times 10^5 \pm 3.5 \times 10^4$ | 85.9 |
| 30 | $11.4 \times 10^5 \pm 2.4 \times 10^5$ | $8.0 \times 10^4 \pm 0.9 \times 10^4$ | 93.0 |

[a] Cells with or without a final concentration of 100 ug per ml HRP were suspended in 0.025M MES buffer, pH 5.2. Aliquots were withdrawn at the indicated times and were plated onto yeast morphology agar.

[b] Colony-forming units per ml were determined in duplicate after 48 hours incubation at 37°C.

[c] Expressed as a percentage of the control.

The effects of the HRP on Streptococcus mutans are shown in Table 6. The results clearly demonstrate the bacteriocidal activity of both the HRP and lysozyme (HEWL) for S. mutans SB. After 1 or 2 hours preincubation in MES buffer, pH 5.2, with these molecules, bacterial growth was inhibited progressively after 24 hours with increasing HRP concentrations and with a fixed concentration of HEWL (Hen egg white lysozyme). For the HRP, this 24 hour bacteriostatic effect correlated with a loss of colony forming units and was dependent on HRP concentration (Table 6). At 50 $\mu$g HRP per ml (Table 6) in the preincubation mixture corresponding to a final concentration of 1 ug HRP per ml in the growth media, approximately 80% of the cells lost their viability. At 250 $\mu$g HRP per ml (Table 6), complete loss of viability was observed under the experimental conditions. After further incubation to a period of 48 hours in the growth media, all cells previously exposed to either HRP or HEWL for 1 hour in the MES buffer attained optical densities similar to the controls. However, with a 2 hour exposure to the MES buffer, those cells showing zero turbidity after 24 hours continued to show the zero turbidity after the 48 hour period of growth and again did not undergo cell division on solid media (Table 6). The 2 hour preincubation in the MES also permitted lysozyme exhibited these effects independent of whether or not Todd Hewitt dialysate was used at neutral or acidic pH (data not shown).

When S. mutans GS5 was tested with the HRP, similar effects were noted. Pre-exposure in the MES buffer to 250 $\mu$g HRP per ml led to complete inhibition of cell division demonstrating the bacteriocidal effects of the HRP for this S. mutans strain (data not shown).

Table 6

Effect of HRP Concentration and Preincubation in Acidic Buffer on the
Growth and Viability of S. mutans SB

| HRP Conc. (ug/ml)[a] | 1 hour preincubation | | | 2 hours preincubation | | |
|---|---|---|---|---|---|---|
| | O.D. 675 nm[b] | CFU[c] | Per cent loss of Viability | O.D. 675 nm[b] | CFU[c] | Per cent loss of viability |
| 0 Control | 0.55 (2.0) | $11.4 \times 10^{10}$ | -- | 0.16 (2.0) | $10.2 \times 10^9$ | -- |
| 25 | 0.48 (2.0) | $8.9 \times 10^{10}$ | 22 | 0.03 (2.0) | $8.9 \times 10^9$ | 13 |
| 50 | 0.22 (2.0) | $27.5 \times 10^9$ | 76 | 0.01 (2.0) | $19.0 \times 10^8$ | 81.4 |
| 100 | 0.05 (2.0) | $9.5 \times 10^9$ | 92 | 0.01 (2.0) | $23.5 \times 10^8$ | 77 |
| 250 | 0.015 (2.0) | $41.0 \times 10^7$ | 99.6 | 0 (0) | 0 (0) | 100 (100) |
| 500 | 0.015 (2.0) | $30.5 \times 10^7$ | 99.7 | 0 (0) | 0 (0) | 100 (100) |
| 500 (HEWL) | 0.08 (2.0) | $35.5 \times 10^9$ | 69.0 | 0 (0) | 0 (0) | 100 (100) |

[a] S. mutans SB at $5 \times 10^5$ colony forming units per ml was suspended and preincubated in MES buffer, pH 5.2, at an ionic strength of 0.025 for 1 and 2 hours at 37°C. Preincubation included various concentrations of HRP or 500 µg HEWL per ml and was followed by 50 fold dilution into Todd Hewitt dialysate growth media.

[b] Optical densities were measured after 24 hours and 48 hours (values in parenthesis) incubation at 37°C for both 1 a nd 2 hour preincubations.

[c] Colony forming units were determined in duplicate after 24 hours for both 1 and 2 hour preincubations. They were also determined after 48 hours on those 2 hour preincubation samples showing zero optical density (values in parenthesis).

EXAMPLE III

Studies with HRP 7

a)    Purification

HRP 7 was purified from the mixture of HRP, isolated by Sephadex G 25 chromatography, by high performance liquid chromatography. The mixture of the HRP was applied to a $\mu$ Bondapak $C_{18}$ Reverse Phase Column. Initial elution was carried out for 15 minutes with distilled water containing 0.1 per cent trifluoroacetic acid. This was followed first by a 15 minute lineargradient to 15 per cent aqueous mathanol containing 0.1% trifluoracetic acid, then by a 90 minute lineargradient to 50% aqueous methanol containing 0.1% trifluoroacetic acid, and finally by an isocratic 60 minute elution with 50% aqueous methanol.

b)    Characterization

HRP 7 is eluted as a single peak in the initial trifluoracetic acid elution off the high pressure liquid chromatographic column as shown by cationic polyacrylamide gel electrophoresis. (data not shown). The amino acid composition is listed above (see Table 2). Based on amino acid analysis, the minimum molecular weight of HRP 7 was calculated to be approximately 7,500 daltons.

c)   Inhibition of Growth of C. albicans 18804

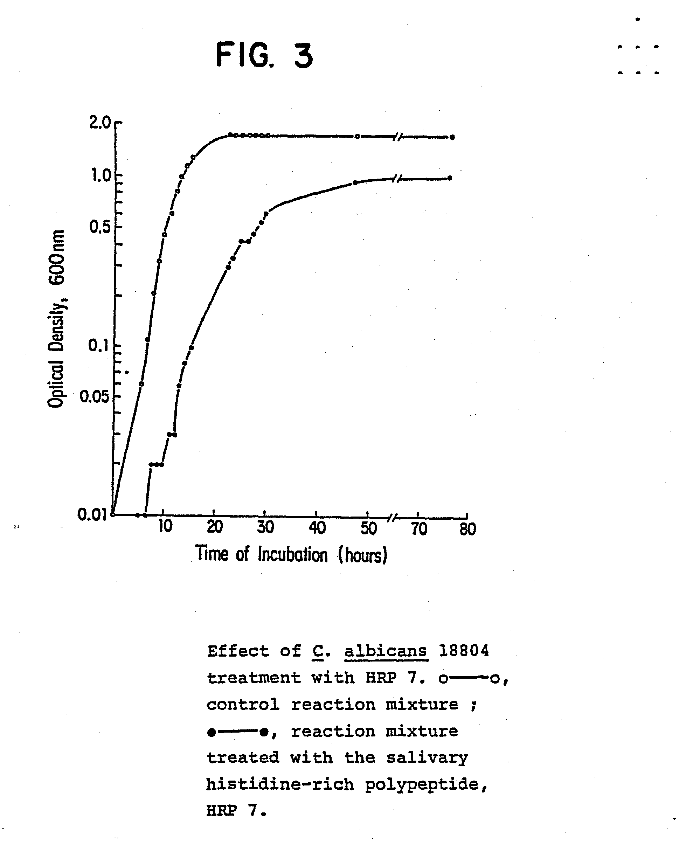

A lyophilized culture of C. albicans 18804 was inoculated at 37°C into yeast synthetic medium (YSM) and
at the late log phase of growth (optical density 600 nm
= 0.95), a second transfer into fresh YSM was made
overnight.  Next morning late log phase cultures were
harvested by centrifugation, washed twice in sterile
distilled water and then suspended in sterile 0.025M
MES buffer, pH 5.3, to an optical density 600 nm =
0.20 ($10^6$ colony forming units per ml).  From this
suspension, 0.1 ml aliquots were transferred to
13 X 100 mm screw cap tubes containing 2.5 ml of YSM
without (controls) or with HRP 7.  Based on optical
density 227 nm absorption, HRP 7 was used at a final
concentration of 80 µg per ml.  However, it should be
noted that HRP 7 may be more active than depicted
below.  The purified material isolated by high performance liquid chromatography was in the trifluoroacetate
form which may inhibit the binding of the histidine
peptide to the negatively-charged fungal cell membrane.
No attempt was made to change the counter anion on the
peptide.  Nevertheless, there was significant inhibition
as noted in Figure 3.

EXAMPLE IV

Effects of Synthetic Oligomers of His-7 and His-4
on Candida Albicans 18804

His-7, containing seven residues of L-histidine,
was a custom synthesis request from Peninsula Laboratories, Belmont, California.  The material was purified
to homogeneity.  Unpurified His-4, containing four
residues of L-histidine, was also obtained from
Peninsula Laboratories.  Both peptides were solubilized in 5 parts of 0.001 M acetic acid and 3.5 parts of 0.025 M MES buffer, pH 5.3. The solutions were filter sterilized through Swinney 0.2 micron GSTF Millipore filters. The final concentration of the peptides were 220 µg per ml which is considerably lower than the 1.5 mg per ml used in the denture experiments.(see below).

A lyophilized culture of C. albicans was inoculated at 37°C into yeast synthetic medium (YSM) snd at the late log phase of growth (optical density 600 nm 0.95), a second transfer into fresh YSM was made overnight. Next morning, late log phase cultures were harvested by centrifugation, washed twice in sterile distilled water and then suspended in sterile 0.025M MES buffer, pH 5.3, to an optical density 600 nm = 0.20 ($10^6$ colony forming units per ml). From this suspension, 0.1 ml aliquots were transferred to 13 x 100 mm screw cap tubes containing 2.5 ml of YSM without (Controls) or with 220 ug per ml of either His-4 or His-7. The growth curve was then followed by measuring the increase in turbidity at 600 nm.

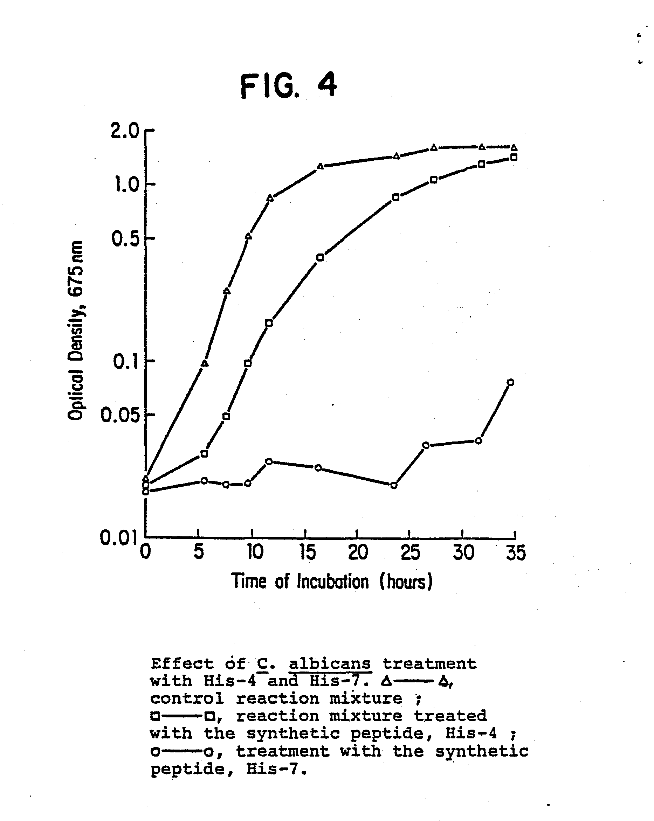

Figure 4 demonstrates that His-7 shows a marked effect on the growth of C. albicans 18804 in suspension culture. After 35 hours of incubation, cells are just beginning to divide. In contrast, His-4 does affect growth but much less than His-7.

## EXAMPLE V

In Vitro Experiments Employing
Denture Acrylic Strips

Denture acrylic was uniformly cut into strips of dimension 1/8 inches x 1/8 inches x 3-1/2 inches. Each strip was attached to the inside of the cap of a screw cap tube (13 x 100mm) such that the bottom of each strip lay 3/8 inches from the bottom of each tube. Tubes containing denture strips were sterilized in the ethylene oxide sterilizer.

a)  Effects of the Synethetic Peptide of Seven Residues of L-Histidine (His-7) on the Proliferation of Candida albicans 18804 on Denture Acrylic Strips

A concentration of 3 mg/ml of His-7 was prepared by solubilizing 25.5 mg of His-7 in a total volume of 8.5 ml consisting of 5 ml of 0.001M acetic acid and 3.5 ml of 0.025M MES buffer, pH 5.3.  The final pH of the solution was 6.0 due to the basic nature of His-7. The solution was sterilized through a Swinney 0.2 micron GSTF Millipore filter.

A lyophilized culture of C. albicans was inoculated at 37°C into yeast synthetic medium (YSM) and at the late log phase of g rowth (optical density 600 nm = 0.95), a second transfer into fresh YSM was made overnight.  Simultaneously, denture acrylic strips were placed overnight at room temperature into tubes containing either 2.5 ml of His-7 or a control tube containing only MES buffer.  Next day, the precoated denture strips were transferred to tubes containing 2.5 ml of the grown up C. albicans (optical density 600 nm = 1.0, corresponding to about $10^7$ colony forming units per ml).  After 2-1/4 hours, the control strip was placed into YSM while His-7 precoated strips were placed into YSM containing 1.5 mg per ml His-7.  Tubes were incubated at 37°C for approximately 24 hours.

The control tube was observed to contain C. albicans in the media indicating that cells had proliferated on the strip, fallen off and multiplied in the media.  The optical density 600 nm read 1.2 after 24 hours incubation. However, there was no growth in the YSM media containing His-7 indicating that His-7 must be affecting proliferation on the denture strip and/or stopping growth of any organisms that fall off into the media.

Both the control tube and the His-7 tubes were then transferred to fresh YSM without any His-7. Tubes were incubated at 37°C and the growth curve was monitored by the increase in optical density at 600 nm.

TABLE 7

Effect of Heptapeptide of L-Histidine on Growth of Candida albicans with Pretreatment of Denture Strips

| Sample | Optical Density at 600 nm - Hours of Incubation | | | | | |
|---|---|---|---|---|---|---|
| | 0 | 9 | 13 | 15 | 27 | 34 |
| Control strip | .01 | .30 | .72 | .98 | 1.5 | 1.75 |
| His-7 strip | .01 | .01 | .02 | .03 | .09 | .50 |
| His-7 strip | .02 | .02 | .03 | .02 | .03 | .05 |

The results shown in Table 7 indicate that either the precoating or the incubation or both with His-7 results in a significant delay in growth at least for 24 hours. Such inhibition would be ideal for the denture patient where the number of Candida could be kept under control.

b)   Effects of Poly-L-Histidine on the Proliferation
of Candida albicans 18804 on Denture Acrylic Strips

Poly-L-histidine is commercially available. We purchased our first batch from Miles Laboratories. The material is prepared by polymerizing the N-carboxy-anhydride of histidine in the presence of a primary or secondary amine. Materials prepared in this manner are heterogeneous in that one obtains a mixture of poly-L-histidine peptides which vary in molecular weight from 5,000 to 15,000 with an average molecular weight of about 10,000.

In this experiment, poly-L-histidine was dissolved in 0.025M MES buffer, pH 5.2 with heating. As in the previous experiment, denture strips were first pretreated with poly-L-histidine (4 mg per ml) overnight prior to incubation with Candida albicans for 2-1/4 hours. Strips were then transferred to YSM without poly-histidine (controls) or to YSM containing polyhistidine at 2 mg per ml. Although there was precipitation of the poly-L-histidine in the YSM, strips were incubated for 24 hours until controls had reached an optical density at 600 nm = 1.2 corresponding to $10^7$ colony-forming units per ml. As in experiment with the L-histidine heptapeptide, strips were then transferred to YSM without poly-L-histidine and the growth curve was followed by monitoring the increase in turbidity.

## Table 8

Effect of Poly-L-Histidine on the Growth of Candida
albicans without Pretreatment of Denture Strips

| Sample | Optical Density at 600 nm ✓ Hours of Incubation | | | | | | |
|--------|------|------|------|------|------|-----|-----|
| | 0 | 12 | 15 | 19 | 31 | 60 | 80 |
| Control Strip | .02 | .46 | .80 | 1.20 | 1.75 | 2.0 | 2.0 |
| Control Strip | .02 | .55 | .85 | 1.25 | 1.75 | 2.0 | 2.0 |
| Poly-His Strip | .02 | .04 | .04 | .05 | .05 | .05 | .05 |
| Poly-His Strip | .02 | .03 | .03 | .03 | .03 | .03 | .03 |

Poly-L-Histine seems to be even more effective than
His-7 (compare Table 8 to Table 7). Growth is inhibited
for 80 hours.

c)   Effect of L-Histidine Heptapeptide without Precoating of Denture Strips

This experiment was similar to the previous two experiments except that there was no precoating of the denture strips.  After incubation of strips in the Candida, a control strip was incubated overnight in the MES-YSM while two additional strips were incubated in MES-YSM containing 1.5 mg per ml of His-7.  Strips were then transferred to YSM without additives and the growth curve was followed as outlined previously.

### Table 9

Effect of a Heptapeptide of L-Histidine on Growth of Candida albicans without Pretreatment of Denture Strips

| Sample | Optical Density at 600 nm - Hours of Incubation | | | | | | |
|---|---|---|---|---|---|---|---|
| | 0 | 7 | 10 | 14.5 | 17.5 | 30 | 36 |
| Control | .01 | .24 | .61 | 1.09 | 1.39 | 1.44 | 1.68 |
| His-7 | .01 | .02 | .01 | .03 | .03 | .03 | .10 |
| His-7 | .01 | .01 | .02 | .06 | .08 | .88 | 1.41 |

Again, notice in Table 9 the variability between strips.  On one His-7 strip, all the Candida seem to have been destroyed while on the other His-7 strip, some organisms have survived such that growth is evident after a long period of time.  However, virtually no growth is seen at 17.5 hr.

Compared to the results with precoating (See Table 7), the effects are similar although precoating of dentures with His-7 may make some difference in the time delay of growth of the fungus.

0149254

## Formulations and Methods of Use

The microbiocidal formulations of the present invention are useful against a variety of organisms found in the oral mucosa, the vaginal mucosa and the urethral mucosa. In the oral cavity problems are caused, in particular, although not exclusively, by Streptococcus mutans and Candida species, particularly, Candida albicans. Vaginitis can be caused by Candida albicans, Trichomonas vaginalis, Haemophilus vaginalis, and various Streptococci and Staphylococci.

In the oral formulations it is desirable to provide for a pH of about pH 5.5. since the histidine peptides are most effective at this range. The vaginal and urethral formulations should be set at a slightly more acidic pH, namely about 4.5. Although this is not optimal from the point of view of effectiveness of the histidine peptides, higher pH's tend to induce pathogenic growth and therefore should be avoided.

The dental formulations when intended as antibacterials should contain between 25 and 500, suitably 50 to 250 milligrams of the peptides per ml.

For antifungal purposes, a slightly higher range is preferable, namely from 25 to 3,000, most suitably 50 to 1,500 milligrams per ml. These formulations apply to the concentrations as they are applied into the mouth.

The vaginal and urethral antibacterial or antifungal agents contain the peptides in between 0.2 to 2% by weight, suitably 0.4 to 1% by weight.

While the invention is in no way considered to be limited thereto, compositions for oral use include toothpaste, mouth sprays, mouthwashes, denture adhesive pastes, denture adhesive powder, denture tablet cleanser and the like and the vaginal and urethral compositions include creams, suppositories, and vaginal deodorant solutions. Specific formulations for the foregoing are set forth in greater detail hereinbelow.

31

EXAMPLE VI

Vaginal Cream:

One of the following:

| | |
|---|---|
| Salivary Histidine-Rich Polypeptides | 0.2 - 2% |
| Poly-L-Histidine | 0.2 - 2% |
| Synthetic Heptapeptide of L-Histidine | 0.2 - 2% |

Allantoin                                           2%

Water Miscible Base to consist of:          75 gram tube

| | |
|---|---|
| Lactose | 20 gm |
| Cetyl Alcohol | 15 gm |
| Stearic Acid | 10 gm |
| Sodium Lauryl Sulfate | 1 gm |
| Glycerin | 35 gm |
| Triethanolamine | 0.2 gm |
| Methyl Paraben | 0.25 gm |
| Propyl Paraben | 0.15 gm |
| $H_2O$ | q.s. to 100 gm |

Buffered with Acetic Acid to pH 4.5

For topical use, gently message cream into the affected and surrounding areas twice daily (morning and evening). For intravaginal use, apply one applicator (5 gm) of cream high into the vaginal vault once or twice daily. Continue until vaginitis is eliminated (usually one week to one month).

EXAMPLE VII

Vaginal Suppositories

One of the following:

| | |
|---|---|
| Salivary Histidine-Rich Polypeptides | 0.2 - 2% |
| Poly-L-Histidine | 0.2 - 2% |
| Synthetic Heptapeptide of L-Histidine | 0.2 - 2% |

Allantoin                                          2%

Water Soluble Base to consist of:          3 gram
                                           suppository

| | |
|---|---|
| Lactose | 10 gm |
| Polyethylene Glycol 400 | 30 gm |
| Polysorbate 80 | 2 gm |
| Polyethylene Glycol 4000 | 24 gm |
| Glycerin | 24 gm |
| Methyl Paraben | 0.25 gm |
| Propyl Paraben | 0.15 gm |
| $H_2O$ | q.s. to 100 gm |

Coloring

Buffered with Acetic Acid to pH 4.5

Insert one suppository high into the vaginal vault
once or twice daily.  Continue until pathogens are
eliminated (usually one week to one month).

## EXAMPLE VIII

Vaginal Deodorant Solution

| | |
|---|---|
| Salivary Histidine-Rich Polypeptides | 0.2 - 2% |
| Poly-L-Histidine | 0.2 - 2% |
| Synthetic Heptapeptide of L-Histidine | 0.2 - 2% |

| | |
|---|---|
| Sodium Perborate | 18 mg |
| Sodium Chloride | 28 mg |
| Sodium Borate | 26 mg |
| Sodium Bicarbonate | 27 mg |
| Menthol | 0.25 mg |
| Thymol | 0.25 mg |
| Methyl Salicylate | 0.50 mg |
| Water | q.s. to 100 ml |

Buffered with Acetic Acid to pH 4.5

Apply daily to the vaginal surfaces.

EXAMPLE IX

Denture Adhesive Paste

One of the following:

| | |
|---|---|
| Salivary Histidine-Rich Polypeptides | 12.5 - 1500 mg |
| Poly-L-Histidine | 12.5 - 1500 mg |
| Synthetic Heptapeptide of L-Histidine | 12.5 - 1500 mg |

| | |
|---|---|
| Carboxymethylcellulose Gum | 32 gm |
| Ethylene Oxide Polymer | 13 gm |
| Petrolatum | 42 gm |
| Liquid Petrolatum | 12 gm |
| Propyl Paraben | 0.05 gm |
| Flavor | 0.05 ml |

Buffer to pH 5.5 with acetic acid

Apply a thin film of antimicrobial paste (approximately 2 gm) onto the surface of each denture and insert into the mouth.  Apply paste to denture after overnight soaking in denture cleanser.

EXAMPLE X

Denture Adhesive Powder

One of the following:

|  |  |
|---|---|
| Salivary Histidine-Rich Polypeptides | 12.5 - 1500 mg |
| Poly-L-Histidine | 12.5 - 1500 mg |
| Synthetic Heptapeptide of L-Histidine | 12.5 - 1500 mg |

|  |  |
|---|---|
| Karaya Gum | 94.6 gm |
| Water-Soluble Ethylene Oxide Polymer | 4.9 gm |
| Calcium Silicate | 0.1 gm |
| Flavor | 0.4 ml |

Buffer to pH 5.5 with acetic acid

Sprinkle antimicrobial powder (approximately 1 to 2 gm) onto the surface of each denture and insert into the mouth. Apply powder to denture after overnight soaking in denture cleanser.

EXAMPLE XI

Denture Tablet Cleanser

Each Tablet

One of the following:

| | |
|---|---|
| Salivary Histidine-Rich Polypeptides | 6 - 720 mg |
| Poly-L-Histidine | 6 - 720 mg |
| Synthetic Heptapeptide of L-Histidine | 6 - 720 mg |

| | |
|---|---|
| Potassium Monopersulfate | 960 mg |
| Sodium Borate Perhydrate | 480 mg |
| Sodium Bicarbonate | 1116 mg |
| Citric Acid | 362 mg |
| Sodium Carbonate | 32 mg |
| Magnesium Stearate | 18 mg |
| Sodium Lauryl Sulfate | 20 mg |
| Peppermint Oil Filter | 2 ml |
| Silica | 14 mg |

Buffer to pH 5.5 with acetic acid

Dissolve each tablet in one denture cup (approximately 8 ounces) of water. Soak denture in antimicrobial denture cleanser overnight prior to reinsertion into the mouth.

EXAMPLE XII

Mouth Spray

One of the following:

| | |
|---|---|
| Salivary Histidine-Rich Polypeptides | 7.5 - 900 mg |
| Poly-L-Histidine | 7.5 - 900 mg |
| Synthetic Heptapeptide of L-Histidine | 7.5 - 900 mg |

| | |
|---|---|
| Peppermint Spirit | 43.2 ml |
| Saccharin Sodium | 0.07 gm |
| Sodium bicarbonate | 0.25 - 3.24 gm |
| Sodium chloride | 0.23 - 1.76 gm |
| Sodium thiocyanate | 0.49 - 2.4 gm |
| Water to | 300 ml. |

Buffer to pH 5.5 with acetic acid

The formulation is utilized as an antibacterial mixture by spraying aliquots of 0.25 to 0.50 ml onto the gingiva and tooth surface of each quadrant between 1 and 3 times a day. For denture stomatitis, apply similar quantities of the spray directly to the denture as an antifungal mixture after overnight soaking in the denture cleanser and prior to reinsertion into the mouth.

38

## EXAMPLE XIII

### Mouthwash Formulation

One of the following:

| | |
|---|---|
| Salivary Histidine-Rich Polypeptides | 25 - 3000 mg |
| Poly-L-Histidine | 25 - 3000 mg |
| Synthetic Heptapeptide of L-Histidine | 25 - 3000 mg |

| | |
|---|---|
| Thymol | 0.5 gm |
| Eucalyptol | 1.0 ml |
| Methyl Salicylate | 0.5 ml |
| Amaranth solution | 14.0 ml |
| Alcohol | 50.0 ml |
| Glycerin | 100.0 ml |
| Water to | 1000.0 ml |

Buffer to pH 5.5 with acetic acid

The formulation is utilized as an antibacterial or antifungal mixture by rinsing the mouth for about 30 - 60 seconds from 1 - 3 times per day with 10 to 15 ml. of undiluted wash.

EXAMPLE XIV

Toothpaste Formulation

One of the following:

| | |
|---|---|
| Salivary Histidine-Rich Polypeptides | 2.5 - 500 mg |
| Poly-L-Histidine | 2.5 - 500 mg |
| Synthetic Heptapeptide of L-Histidine | 2.5 - 500 mg |

| | |
|---|---|
| Carboxymethyl cellulose 120H | 1.8 gm |
| Glycerin | 2.0 ml |
| Propylene glycol | 39.0 ml |
| Purified water | 27.0 ml |
| Methyl paraben | 0.2 gm |
| Saccharin Sodium (50% sol.) | 0.2 ml |
| Peppermint Oil | 0.6 ml |
| Mineral Oil | 2.0 ml |
| Triton X-100 | 5.0 gm |
| Silica | 1.0 gm |

Buffer to pH 5.5 with acetic acid

The formulation is utilized as an antibacterial mixture by cleaning the teeth with about 1 to 2 gm of the paste between 1 and 3 times per day.

CLAIMS:

1.    An anti fungal composition comprising a pharmaceutically acceptable carrier and a fungicidally or fungistatically effective amount of at least one polypeptide containing at least 4 amino acid units and 14 mole % of the amino acid residues of L-Histidine.

2.    A composition of Claim 1 wherein the polypeptide is selected from the group consisting of poly-L-Histidine, tetra thru deca-L Histidine and polypeptides having the structure of histidine-rich polypeptides derived from human saliva having the characteristics of the peptide of Claim 1.

3.    A composition according to Claim 1 wherein the carrier is a topically administerable carrier.

4.    A composition of Claim 3 wherein the carrier is a carrier adapted for application to mucosa wherein the mucosa is oral, vaginal or urethral mucosa.

5.    A composition of Claim 4 wherein the carrier is a dentifrice, a mouthwash, a mouth spray, a denture wash, or a denture adhesive.

6.    A composition of Claim 5 wherein the carrier is a vaginal cream, a vaginal deodorant solution or a vaginal suppository.

7.    A composition of Claim 4 wherein the carrier is a polymer having the polypeptide incorporated therein in a pharmacologically effectively releasable manner.

8.    A composition in accordance with Claim 7 wherein the polymer is a water swellable polymer.

9.    An  oral   antifungal  composition   of  Claim  1 comprising between   25 and 3,000 ug/ml. of the polypeptide and an orally acceptable carrier therefore.

10.    An  antivaginitically  effective  composition of Claim  1 comprising  between 0.2  and 2%  by weight of the polypeptide in a vaginally acceptable carrier.

# FIG. I

Effect of addition of the histidine-rich polypeptides to Candida albicans 18804 during the growth cycle. See text for details.

●——●, normal growth curve of C. albicans 18804 in yeast synthetic media; O•——O, addition of 50 µg HRP per ml at time on growth curve designated by the arrow (• ——→); Δ••••Δ, 50 µg HRP per ml at time indicated by arrow (••••>); ▲••••▲, 250 µg HRP per ml at time indicated by arrow (••••>);□ ——□, 50 µg HRP per ml at time indicated by arrow (——>); and■ ——■, 250 µg HRP per ml at time indicated by arrow (——>).

## FIG. 2

Comparison of the inhibition of viability of three strains of <u>Candida</u> <u>albicans</u> by the histidine-rich polypeptides under non-growing conditions. See text for details.  0——0, <u>C</u>. <u>albicans</u> 18804, S.E. - see Table 2; ▲——▲, <u>C</u>. <u>albicans</u> 28517, S.E. ± 15.96%, error range 7.79 to 35.14%; and ●——●, <u>C</u>. <u>albicans</u> 28815, S.E. ± 13.62% error range 7.35 to 24.53%.

# FIG. 3

Effect of C. albicans 18804 treatment with HRP 7. o———o, control reaction mixture ; ●———●, reaction mixture treated with the salivary histidine-rich polypeptide, HRP 7.

# FIG. 4

Effect of C. albicans treatment
with His-4 and His-7. Δ———Δ,
control reaction mixture ;
□———□, reaction mixture treated
with the synthetic peptide, His-4 ;
o———o, treatment with the synthetic
peptide, His-7.